# EUROPEAN PATENT SPECIFICATION

(11) **EP 4 014 765 B1**
(45) Date of publication and mention of the grant of the patent: **06.03.2024**
(21) Application number: 20885611.2
(22) Date of filing: 17.09.2020
(51) Int. Cl.: A24F 40/51, A24F 40/53, A24F 40/65, A61M 15/06

(54) **ELECTRONIC VAPORIZING DEVICE CHIP WITH AIR PRESSURE SENSING UNIT AND WORKING METHOD THEREFOR**
ELEKTRONISCHER ZERSTÄUBERCHIP MIT LUFTDRUCKSENSOREINHEIT UND BETRIEBSVERFAHREN DAFÜR
PUCE D'APPAREIL D'ATOMISATION ÉLECTRONIQUE COMPRENANT UNE UNITÉ DE DÉTECTION DE PRESSION D'AIR ET SON PROCÉDÉ DE FONCTIONNEMENT

(30) Priority: 05.11.2019 CN 201911071157
(43) Date of publication of application: 22.06.2022
(73) Proprietor: Shenzhen Happy Vaping Technology Limited, Shenzhen, Guangdong 518104 (CN)
(72) Inventor: LIN, Guangrong, Shenzhen, Guangdong 518104 (CN); ZHANG, Xiyong, Shenzhen, Guangdong 518104 (CN); ZHENG, Xianbin, Shenzhen, Guangdong 518104 (CN)
(74) Representative: Meyer, Thorsten
(86) International application number: PCT/CN2020/115735
(87) International publication number: WO 2021/088523

(56) References cited:
- EP-A1- 3 151 690
- EP-B1- 3 308 661
- CN-A- 103 622 160
- CN-A- 106 579 567
- CN-A- 106 579 567
- CN-A- 108 338 416
- CN-A- 109 043 670
- CN-A- 109 924 548
- CN-A- 109 924 548
- CN-A- 109 924 548
- CN-A- 110 209 441
- CN-A- 110 209 441
- CN-A- 110 353 318
- CN-A- 110 693 094
- CN-U- 204 217 894
- CN-U- 206 574 788
- CN-U- 211 323 077
- US-A1- 2019 204 126

## Description

### TECHNICAL FIELD

The present disclosure relates to a technical field of the electronic vaporizing device, and more particularly, the present disclosure relates to an electronic vaporizing device chip with an air pressure sensing unit and a working method of the electronic vaporizing device chip with an air pressure sensing unit.

### BACKGROUND

The existing vaporized electronic cigarettes are usually heated by the vaporizer to vaporize the electronic cigarette liquid to generate vapor for the smoker to use. Since the electronic cigarette liquid does not contain the cigarette tar, the harm to the human body is avoided. Thus, the electronic cigarettes are widely used and gradually replace tobacco cigarettes.

The Chinese patent with application No. 201910269123.8, publication No. 109924548A, entitled "vaporizing device capable of controlling administration amount, and control method for same" discloses a vaporizing device capable of controlling administration amount. The vaporizing device comprises a battery, a control circuit board, a heating resistor, a vaporizing passage, a mouth piece, a detecting air passage, and an air pressure sensor. The control circuit board is arranged with a microcontroller, an air flow quantity calculating unit, an energy statistics unit, an energy and vaporizing amount conversion unit, and a power control unit, which are electrically connected with each other. The air flow quantity calculating unit calculates corresponding air flow quantity based on the modeling volume of the detecting air passage and a change of suction pressure. The power control unit controls a power output to the heating resistor based on the air flow quantity. The energy statistics unit calculates output energy based on the output power and time. The energy and vaporizing amount conversion unit calculates corresponding value of aerosol intake from the energy. The patent discloses that the change of the suction pressure is detected by the air pressure sensor, and then the change of the suction pressure is converted into the air flow quantity by the air flow quantity calculation unit, and finally the purpose of limiting the intake of aerosol is achieved.

In the above patent, the air pressure sensor and the air flow quantity calculation unit belong to separate components and circuit units respectively, the circuit structure is complex, the volume thereof is large, the signal standard is not uniform, and the conversion takes a long time, so data transmission between the two has disadvantages of a slow response and a large error, and at the same time, a control accuracy is reduced.

Application with publication number of CN108338416A disclosed an inner core heating and smoking system comprises a heater, which comprises: the heater shell, the holding chamber in the heater shell, the heating core in the holding chamber, and the micro heater on the outer wall of the heating core. The inner core heating and smoking system disclosed by the invention uses a micro heater arranged on the outer wall of the heating core for heating. Compared with the prior art, the inner core heating and smoking system can transmit heat from the heating core to the smoke body placed inside the holding bin quickly, thus speeding up the heating of the smoke body, effectively improving the heating efficiency, and shortening the waiting time for the first suction.

Application with publication number of EP3151690A1 disclosed an electronic vapour provision system includes a vaporiser for vaporising liquid for inhalation by a user of the electronic vapour provision system; a power supply comprising a cell or battery for supplying power to the vaporiser; a sensor for measuring the rate of airflow through the electronic vapour provision system as a result of the inhalation by the user; and a control unit for controlling the power supplied to the vaporiser based on a cumulative airflow for this inhalation by the user, wherein the cumulative airflow is determined based on the measurements of airflow rate by the sensor. Such a system allows the user control over the amount of vapourised liquid obtained in a given inhalation based on the cumulative airflow for the given inhalation.

Application with publication number of US20190204126A1 disclosed a sensor apparatus may include a channel structure configured to couple with an external element and a fluid conduit, such that the channel structure may receive a fluid, at least partially drawn through the external element from an ambient environment, and direct the fluid through the fluid conduit. A sensor may generate sensor data indicating a flow rate of the fluid through the fluid conduit based on monitoring a variation in a pressure at a location in hydrodynamic contact with the fluid conduit and in relation to an ambient pressure of the ambient environment. The sensor apparatus may enable generation of improved topography information associated with flows of fluid drawn from the external element based on measuring a local pressure at the location in hydrodynamic contact with the fluid conduit and determining the ambient pressure based on monitoring the local pressure over time.

### SUMMARY

### TECHNICAL PROBLEM

An object of the present disclosure is to provide an electronic vaporizing device chip with air pressure sensing unit and working method thereof in order to overcome the deficiencies of the above-mentioned technology. The electronic vaporizing device chip with an air pressure sensing unit combines the air pressure sensing function and the air flow quantity calculating function into one.

### TECHNICAL SOLUTION

The technical solution of the present disclosure is achieved as follows, an electronic vaporizing device chip with an air pressure sensing unit, comprising an air pressure sensing unit, a control unit, a plurality of auxiliary resistors, a capacitor, and a plurality of pins, wherein the air pressure sensing unit, the control unit, the plurality of auxiliary resistors, the capacitor, and the plurality of pins are electrically connected. The control unit comprises a memory, a control logic module, an air pressure sensing unit driving module, a signal amplification module, an analog-to-digital conversion module, a data processing and calibration module, a communication interface module, and an enabling signal input control circuit. The air pressure sensing unit is configured to detect an air pressure generated inside the electronic vaporizing device when suction, the memory is configured to store a relevant parameter, the control logic module is configured for a logic control of an internal circuit of the control unit, the air pressure sensing unit driving module is configured to drive the air pressure sensing unit to work, the signal amplification module is configured to amplify and then transmit an air pressure analog signal detected by the air pressure sensing unit to the analog-to-digital conversion module, the analog-to-digital conversion module converts the air pressure analog signal into an air pressure digital signal, the data processing and calibration module processes and calibrates the air pressure digital signal and then converts it into an air flow quantity digital signal, the communication interface module is configured for a communication connection with an external component, and the enabling signal input control circuit is configured to receive an external enabling signal and control the communication interface module to work. The electronic vaporizing device chip further comprises a temperature sensing unit for detecting temperature of the electronic vaporizing device chip, wherein the control unit further comprises a circuit switching module for switching the air pressure analog signal detected by the air pressure sensing unit and a temperature analog signal detected by the temperature sensing unit, and then transmitting the switched air pressure analog signal or the switched temperature analog signal to the analog-to-digital conversion module in turn, and the analog-to-digital conversion module may be capable of convert the temperature analog signal into a temperature digital signal.

Preferably, the control unit is inactive in a standby state, the air pressure sensing unit is configured to have a patrol detection for the air pressure, and the control unit immediately enters a working state once a change of the air pressure is detected.

Preferably, the plurality of pins comprise at least a power supply pin, a ground pin, an activation signal output pin, an internal data output pin, an external data input pin, a clock signal pin, and an enabling signal input pin. The activation signal output pin serves to output an activation signal generated by the control unit to an external controller so as to activate the electronic vaporizing device. The internal data output pin, the external data input pin, and the clock signal pin are connected to the communication interface module, the internal data output pin serves to output an internal data to the external controller, and the external data input pin serves to input external data, the clock signal pin serves to input a clock signal to read and write data, and the enabling signal input pin serves to input an enabling signal to control an output of the internal data and an input of the external data.

Preferably, an air pressure change threshold value is preset in the memory, and the control unit is configured to generate the activation signal in a condition that the control unit determines that a change value of the air pressure digital signal reaches the preset air pressure change threshold value.

Preferably, the control unit further comprises an output control module electrically connected with the data processing and calibration module, the output control module is configured to generate a PWM output control signal based on the air flow quantity digital signal for controlling power of the electronic vaporizing device.

Preferably, the pins further comprise a PWM output control signal pin, and the PWM output control signal pin serves to output the PWM output control signal generated by the output control module.

Preferably, the control unit further comprises an on-chip oscillator for providing a frequency signal for the control unit during working.

Preferably, the control unit further comprises a real-time clock module for timing.

Another technical solution of the present disclosure is a working method of an electronic vaporizing device chip with an air pressure sensing unit, wherein the working method comprises steps of:
(1) initializing a relevant parameter when the chip is powered;
(2) activating an air pressure sensing unit and a temperature sensing unit when the chip is in a standby state, to simultaneously go to steps (3) and (4);
(3) determining whether the air pressure sensing unit detects a change of an air pressure analog signal of the electronic vaporizing device, if yes, go to step (5), if no, return to step (2);
(4) detecting a temperature analog signal of an internal temperature of the chip by the temperature sensing unit;
(5) switching the air pressure analog signal and the temperature analog signal in turn by a circuit switching module, if being switched to the air pressure analog signal, go to step (6); if being switched to the temperature analog signal, go to step (12);
(6) amplifying the air pressure analog signal by a signal amplification module and converting the amplified air pressure analog signal into an air pressure digital signal by an analog-to-digital conversion module;
(7) processing and calibrating the air pressure digital signal by a data processing and calibration module and converting the processed and calibrated air pressure digital signal into a corresponding air flow quantity digital signal;
(8) by means of a control logic module, determining whether a change value of the air pressure digital signal reaches a set air pressure change threshold value, if yes, simultaneously go to steps (9), (10), and (12), if no, return to step (2);
(9) generating an activation signal by a control unit and outputting the activation signal to an external controller through an activation signal output terminal to activate the electronic vaporizing device;
(10) determining whether the air flow quantity digital signal reaches a set minimum air flow quantity threshold value, if yes, go to next step, if no, return to step (2);
(11) generating and outputting a PWM output control signal by an output control module, then returning to step (2);
(12) determining whether an enabling signal input control circuit receives an enabling signal and whether a communication interface module receives a data transmission request signal, if both yes, go to next step, if no, return to step (2);
(13) outputting the air flow quantity digital signal to the external controller through an internal data output pin by the communication interface module, then returning to step (2);
(14) amplifying the temperature analog signal by the signal amplification module and converting the amplified temperature analog signal into a temperature digital signal by the analog-to-digital conversion module;
(15) by means of the control logic module, determining whether the temperature digital signal reaches a set temperature threshold value, if yes, go to next step, if no, return to step (2); and
(16) inactivating the chip and providing high temperature or low temperature protection.

### BENEFICIAL EFFECT

The electronic vaporizing device chip with an air pressure sensing unit has a built-in air pressure sensing unit and a control unit. The control unit directly amplifies, converts, and calibrates an air pressure signal detected by the air pressure sensing unit inside the chip through a control logic module, a signal amplification module, an analog-to-digital conversion module, and a data processing and calibration module, and outputs an air flow quantity signal. In this way, a built-in air pressure signal acquisition circuit and a high-accuracy analog-to-digital converter allow an accurate acquisition of the air pressure signal, the built-in control unit allows an accurate calibration and compensation of the signal, and a built-in algorithm allows an accurate conversion of the pressure to the air flow. Therefore, a data transmission response can be fast, and the air pressure signal can be accurately converted inside the chip, which will greatly improve a control accuracy of a vaporizing amount of the electronic vaporizing device. At the same time, a customizable air pressure change threshold value is provided, and an activation signal is given when a change of the air pressure is greater than a set threshold value, in such a case, a user's vaping action may be accurately judged and a probability of a false activation of the electronic vaporizing device may be reduced. In addition, the chip has a high integration level, so that there are few external components, and the chip has high work efficiency, with less amount of heat being generated. Due to the small size, the chip can be very easily installed on a circuit board of the electronic vaporizing device which has very small space.

### BRIEF DESCRIPTION OF DRAWINGS

FIG. 1 is a first internal circuit structure diagram of an electronic vaporizing device chip according to an embodiment of the present disclosure;
FIG. 2 is a second internal circuit structure diagram of an electronic vaporizing device chip according to an embodiment of the present disclosure;
FIG. 3 is a first flowchart of a working method of an electronic vaporizing device chip according to an embodiment of the present disclosure;
FIG. 4 is a second flowchart of a working method of an electronic vaporizing device chip according to an embodiment of the present disclosure.

### DETAILED DESCRIPTION

In order to make the objects, technical solutions, and advantages of the present disclosure clearer, the present disclosure will be further described in detail below with reference to the accompanying drawings and embodiments. It should be understood that the specific embodiments described herein are merely used for explaining the present disclosure, and are not intended to limit the present disclosure.

The electronic vaporizing device mentioned in the present disclosure is an vaporizing device or an vaporizer that heats and evaporates a liquid substance into a vaporous mist-like substance. The electronic vaporizing device can not only be used for electronic cigarettes to vaporize electronic cigarette liquid to generate vapor, but also can be used in the medical field, that is, a liquid in which drugs are dissolved can be heated and evaporated into a mist-like substance for patients to vape, so as to achieve the purpose of treatment.

### Embodiment 1:

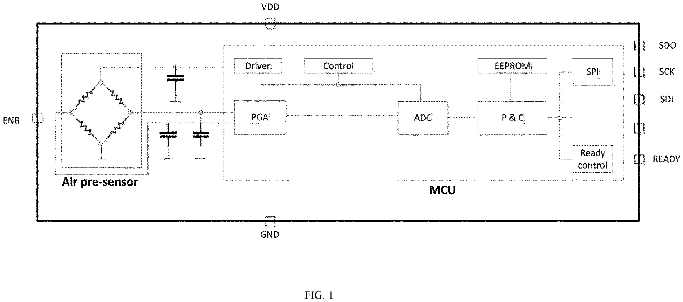

As shown in FIG. 1, an electronic vaporizing device chip with an air pressure sensing unit of the present disclosure, an internal package of which comprising an air pressure sensing unit Airpre-sensor, a control unit MCU, a plurality of auxiliary resistors (not shown in the figure), a capacitor C, and a plurality of pins provided on sides of the chip, wherein the air pressure sensing unit Airpre-sensor, the control unit MCU, the plurality of auxiliary resistors (not shown in the figure), the capacitor C, and the plurality of pins provided on sides of the chip are electrically connected. The control unit MCU comprises a memory EEPROM, a control logic module Control, an air pressure sensing unit driving module Driver, a signal amplification module PGA, an analog-to-digital conversion module ADC, a data processing and calibration module P&C, a communication interface module SPI, and an enabling signal input control circuit Ready control. The air pressure sensing unit Airpre-sensor is configured to detect an air pressure generated inside the electronic vaporizing device when suction. The suction generally generates a negative pressure, and the air pressure sensing unit Airpre-sensor obtains an air pressure analog signal after detecting.

The memory EEPROM is configured to store a set relevant parameter and a relevant parameter generated during working, the control logic module Control is configured for a logic control, an analysis, and a judgment of an internal circuit of the control unit MCU, the air pressure sensing unit driving module Driver is configured to assist and drive the air pressure sensing unit Airpre-sensor to work, the signal amplification module PGA is configured to amplify the air pressure analog signal detected by the air pressure sensing unit Airpre-sensor and transmit it to the analog-to-digital conversion module ADC, the analog-to-digital conversion module ADC converts the air pressure analog signal into an air pressure digital signal, and the data processing and calibration module P&C processes and calibrates the air pressure digital signal and then converts it into an air flow quantity digital signal. Since the external air pressure is not stable and constant, calibration is needed to avoid errors.

The communication interface module SPI and the enabling signal input control circuit Readycontrol connect some of the plurality of the pins. The communication interface module SPI is used for a communication connection with an external component and input or output a data signal, and the enabling signal input control circuit Readycontrol is configured to receive an external enabling signal and control the communication interface module SPI to work, that is, when the enabling signal is received, the enabling signal input control circuit Readycontrol enable the communication interface module SPI to output data or input data.

In order to reduce an energy consumption of the electronic vaporizing device during standby, the chip of the present disclosure is also provided with a standby state. The control unit MCU is inactive in the standby state, and the air pressure sensing unit Airpre-sensor is configured to have a patrol detection for the air pressure, so as to save power. When the air pressure sensing unit Airpre-sensor detects that the air pressure changes, the control unit MCU immediately enters a working state. When a suction occurs, a negative pressure will be generated inside the electronic vaporizing device, that is, the pressure will change. When the air pressure sensing unit Airpre-sensor detects a change of the air pressure analog signal inside the electronic vaporizing device, the control unit MCU is woken up, powered and enters the working state.

As shown in FIG. 1, the pins of the chip comprise a power supply pin VDD, a ground pin GND, an activation signal output pin ENB, an internal data output pin SDO, an external data input pin SDI, a clock signal pin SCK, and an enabling signal input pin READY The activation signal output pin ENB serves to output an activation signal generated by the control unit MCU to an external controller (not shown in the figure) in order to activate the electronic vaporizing device. The internal data output pin SDO, the external data input pin SDI, and the clock pin SCK are connected to the communication interface module SPI. The internal data output pin SDO serves to output internal data to the external controller, the external data input pin SDI serves to input external data, and the clock signal pin SCK serves to input a clock signal to read and write data. The enabling signal input pin READY serves to input the enabling signal to control the internal data output and the external data input.

As shown in FIG. 1, an air pressure change threshold value is preset in the memory EEPROM, and the control unit MCU is configured to generate the activation signal in a condition that the control unit MCU determines that a change value of the air pressure digital signal reaches the set air pressure change threshold value. That is, when the control unit MCU determines that the change value of the air pressure digital signal reaches the set air pressure change threshold value, the control unit MCU generates the activation signal to be output to the external controller, thereby activating the electronic vaporizing device. Under normal circumstances, a change of an internal pressure of the electronic vaporizing device unintentionally caused by a user, and a change of the external air pressure may falsely trigger the electronic vaporizing device to enter the working state. In order to avoid this situation, it is necessary to preset the air pressure change threshold value. When a value of the change of the internal pressure of the electronic vaporizing device exceeds the preset air pressure change threshold value, it is determined that the electronic vaporizing device needs to be started to work. Otherwise, even if there is a slight change of the air pressure, the electronic vaporizing device will not be activated by false triggering.

The chip of the embodiment is characterized in that it is packaged with an air pressure sensing unit Airpre-sensor, a control unit MCU, and a plurality of peripheral auxiliary resistors, and an auxiliary capacitor. The air pressure sensing unit Airpre-sensor and the control unit MCU can be set as two built-in bare chips. The air pressure sensing unit Airpre-sensor is a sensor that uses a pressure sensitive element, that is, a bridge pressure sensor to detect a change value of an air pressure, and the change value of the air pressure could be converted to corresponding air flow quantity. A piezoresistive pressure sensor is integrated into a single chip of the embodiment, and a sensor signal conditioning and transmission circuit with the control unit MCU as the core is integrated into the chip as well, and an SPI interface is used for output. The chip of the embodiment detects the air flow quantity by detecting a change of the air pressure, a built-in signal acquisition circuit and a high-accuracy analog-to-digital converter allow an accurate acquisition of a signal of a sensing unit, and the built-in MCU allows a calibration and compensation of the signal, a built-in algorithm allows a conversion of pressure to airflow, and measurement data is provided synchronously through the SPI interface and sent to an external component. The chip has high flexibility, and algorithms and functions thereof can be extended.

### Embodiment 2:

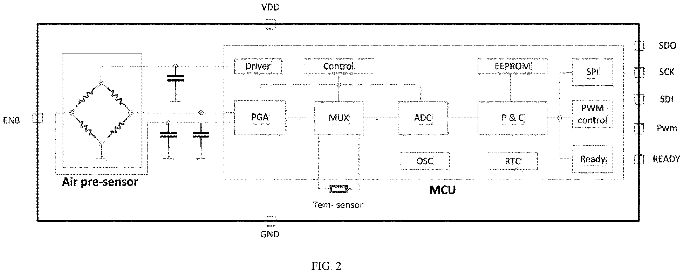

As shown in FIG. 2, on the basis of the foregoing embodiment 1, the electronic vaporizing device chip of this embodiment comprises, in addition to the circuit structural components described in embodiment 1, a temperature sensing unit Tem-sensor for detecting a temperature of the electronic vaporizing device chip.The control unit MCU also comprises a circuit switching module MUX, which is configured to switch the air pressure analog signal detected by the air pressure sensing unit Airpre-sensor and a temperature analog signal detected by the temperature sensing unit Tem-sensor in turn, and then transmit the switched air pressure analog signal or the switched temperature analog signal to the analog-to-digital conversion module ADC in turn. The analog-to-digital conversion module ADC can also convert the temperature analog signal into a temperature digital signals. The purpose of providing the temperature sensing unit Tem-sensor is to detect a temperature of the chip during working, prevent abnormal temperature caused by the chip working under high load or in a high temperature or low temperature environment, and avoid chip damage and failure.

As shown in FIG. 2, the control unit MCU further comprises an output control module PWMcontrol electrically connected with the data processing and calibration module P&C, and the output control module PWMcontrol is configured to generate a PWM output control signal. The pins also comprise a PWM output control signal pin PWM, and the PWM output control signal pin serves to output a PWM output control signal generated by the output control module PWMcontrol. The output control module PWMcontrol is configured to generate the PWM output control signal based on the air flow quantity digital signal for controlling power of the electronic vaporizing device.

As shown in FIG. 2, the control unit MCU further comprises an on-chip oscillator OSC for providing a frequency signal when the control unit MCU works. The control unit MCU also comprises a real-time clock module RTC for timing and reading time.

The chip of the embodiment is characterized in that it is packaged with an air pressure sensing unit Airpre-sensor, a control unit MCU, and a plurality of peripheral auxiliary resistors, and an auxiliary capacitor. The air pressure sensing unit Airpre-sensor and the control unit MCU can be set as two built-in bare chips. The air pressure sensing unit Airpre-sensor is a sensor that uses a pressure sensitive element, that is, a bridge pressure sensor to detect a change value of an air pressure, and the change value of the air pressure could be converted to corresponding air flow quantity. A piezoresistive pressure sensor and a temperature sensor are integrated into a single chip of the embodiment, an air pressure sensing unit signal conditioning and transmission circuit with the control unit MCU as the core is integrated into the chip as well, an SPI interface is used for output, and a PWM signal output is also supported. The chip of the embodiment detects the air flow quantity by detecting a change of the air pressure, a built-in signal acquisition circuit and a high-accuracy analog-to-digital converter allow an accurate acquisition of a signal of a sensing unit, and the built-in MCU allows a calibration and compensation of the signal, a built-in algorithm allows a conversion of pressure to airflow. At the same time, a customizable air flow quantity threshold value is provided, a PWM control signal is given when the air flow quantity is greater than a set minimum air flow quantity threshold value, and measurement data is provided synchronously through the SPI interface and sent to an external component. The chip has high flexibility, and algorithms and functions can be extended.

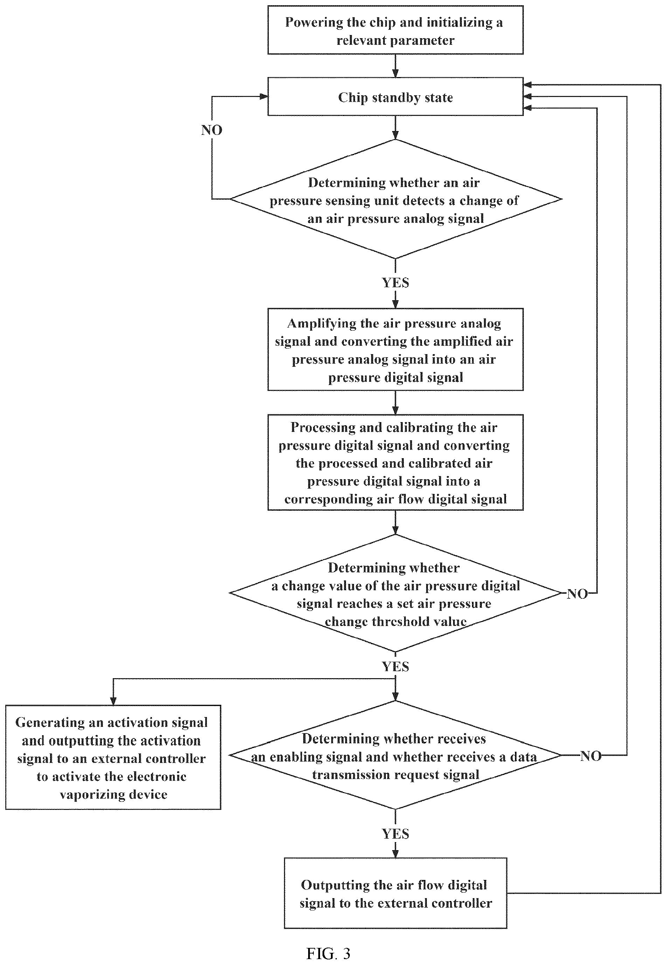

Embodiment 1 of working methods of the chip:
As shown in FIG. 3, a working method of an electronic vaporizing device chip with an air pressure sensing unit, wherein the working method comprises steps of:
(1) initializing a relevant parameter when the chip is powered;
(2) activating an air pressure sensing unit when the chip is in a standby state and a control unit is inactive;
(3) determining whether the air pressure sensing unit detects a change of an air pressure analog signal of the electronic vaporizing device, that is, detecting whether the electronic vaporizing device has an action of suction, if yes, go to next step, if no, return to previous step;
(4) activating the control unit, and amplifying the air pressure analog signal by a signal amplification module and converting the amplified air pressure analog signal into an air pressure digital signal by an analog-to-digital conversion module;
(5) processing and calibrating the air pressure digital signal by a data processing and calibration module into an accurate air pressure digital signal, and converting the accurate air pressure digital signal into a corresponding air flow quantity digital signal;
(6) by means of a control logic module, determining whether a change value of the air pressure digital signal reaches a set air pressure change threshold value, that is, determining whether the action of suction is caused by a false trigger action, if yes, that is, the action of suction is not caused by the false trigger action, simultaneously go to steps (7) and (8), if no, that is, the action of suction may be caused by the false trigger action, return to step (2);
(7) generating an activation signal by a control unit and outputting the activation signal to an external controller through an activation signal output terminal to activate the electronic vaporizing device;
(8) determining whether an enabling signal input control circuit receives an enabling signal and whether a communication interface module receives a data transmission request signal, if both yes, go to next step, if no, return to step (2); and
(9) outputting the air flow quantity digital signal to the external controller through an internal data output pin by the communication interface module, and then returning to step (2).

For the names of the components or modules involved in the above steps, referring to Embodiment 1 and FIG. 1, in the above steps, the air pressure sensing unit continuously detects whether the air pressure changes.

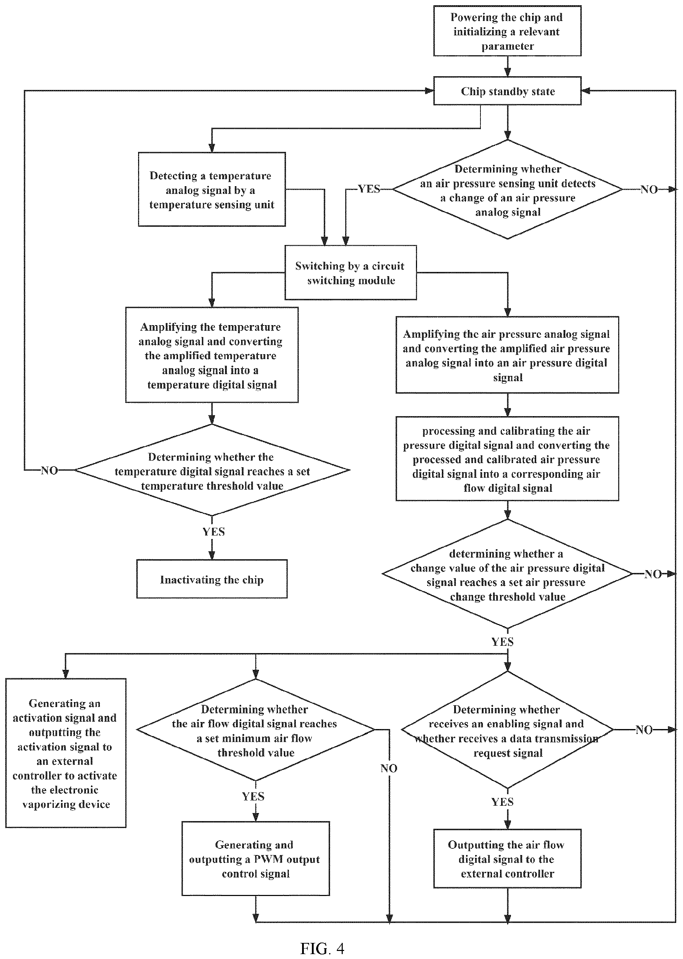

Embodiment 2 of the working methods of the chip:
As shown in FIG. 4, a working method of an electronic vaporizing device chip with an air pressure sensing unit, wherein the working method comprises steps of:
(1) initializing a relevant parameter when the chip is powered;
(2) activating an air pressure sensing unit and a temperature sensing unit when the chip is in a standby state, to simultaneously go to steps (3) and (4);
(3) determining whether the air pressure sensing unit detects a change of an air pressure analog signal of the electronic vaporizing device, that is, detecting whether the electronic vaporizing device has an action of suction, if yes, go to step (5), if no, return to step (2);
(4) detecting a temperature analog signal of an internal temperature of the chip by the temperature sensing unit;
(5) switching the air pressure analog signal and the temperature analog signal in turn by a circuit switching module, if being switched to the air pressure analog signal, go to step (6); if being switched to the temperature analog signal, go to step (12);
(6) amplifying the air pressure analog signal by a signal amplification module and converting the amplified air pressure analog signal into an air pressure digital signal by an analog-to-digital conversion module;
(7) processing and calibrating the air pressure digital signal by a data processing and calibration module into an accurate air pressure digital signal, and converting the accurate air pressure digital signal into a corresponding air flow quantity digital signal;
(8) by means of a control logic module, determining whether a change value of the air pressure digital signal reaches a set air pressure change threshold value, that is, determining whether the action of suction is caused by a false trigger action, if yes, that is, the action of suction is not caused by the false trigger action, simultaneously go to steps (9), (10), and (12), if no, that is, the action of suction may be caused by the false trigger action, return to step (2);
(9) generating an activation signal by a control unit and outputting the activation signal to an external controller through an activation signal output terminal to activate the electronic vaporizing device;
(10) determining whether the air flow quantity digital signal reaches a set minimum air flow quantity threshold value, that is, determining whether the action of suction is caused by a false trigger action, if yes, that is, the action of suction is not caused by the false trigger action, go to next step, if no, that is, the action of suction may be caused by the false trigger action, return to step (2);
(11) generating and outputting a PWM output control signal by an output control module, then returning to step (2);
(12) determining whether an enabling signal input control circuit receives an enabling signal and whether a communication interface module receives a data transmission request signal, if both yes, go to next step, if no, return to step (2);
(13) outputting the air flow quantity digital signal to the external controller through an internal data output pin by the communication interface module, then returning to step (2);
(14) amplifying the temperature analog signal by the signal amplification module and converting the amplified temperature analog signal into a temperature digital signal by the analog-to-digital conversion module;
(15) by means of the control logic module, determining whether the temperature digital signal reaches a set temperature threshold value, if yes, go to next step, if no, return to step (2); and
(16) inactivating the chip and providing high temperature or low temperature protection, the air pressure sensing unit and the control unit are both inactive at this time, and after a time of being protected from high temperature or low temperature is over, reactivating the chip.

For the names of the components or modules involved in the above steps, referring to Embodiment 2 and FIG. 2, in the above steps, the air pressure sensing unit continuously detects whether the air pressure changes.

Industrial applicability:
The above descriptions are only preferred embodiments of the present invention, and changes and modifications may be made within the scope of the appended claims.

## Claims

1. An electronic vaporizing device chip with an air pressure sensing unit, comprising an air pressure sensing unit, a control unit, a plurality of auxiliary resistors, a capacitor, and a plurality of pins, wherein the air pressure sensing unit, the control unit, the plurality of auxiliary resistors, the capacitor, and the plurality of pins are electrically connected, **characterized in that** the control unit comprises a memory, a control logic module, an air pressure sensing unit driving module, a signal amplification module, an analog-to-digital conversion module, a data processing and calibration module, a communication interface module, and an enabling signal input control circuit, the air pressure sensing unit is configured to detect an air pressure generated inside the electronic vaporizing device during suction, the memory is configured to store a relevant parameter, the control logic module is configured for a logic control of an internal circuit of the control unit, the air pressure sensing unit driving module is configured to drive the air pressure sensing unit to work, the signal amplification module is configured to amplify and then transmit an air pressure analog signal detected by the air pressure sensing unit to the analog-to-digital conversion module, the analog-to-digital conversion module is configured to convert the air pressure analog signal into an air pressure digital signal, the data processing and calibration module is configured to process and calibrate the air pressure digital signal, and then convert the air pressure digital signal into an air flow quantity digital signal, the communication interface module is configured for a communication connection with an external component, and the enabling signal input control circuit is configured to receive an external enabling signal and control the communication interface module to work, and the electronic vaporizing device chip further comprises a temperature sensing unit for detecting temperature of the electronic vaporizing device chip, wherein the control unit further comprises a circuit switching module for switching the air pressure analog signal detected by the air pressure sensing unit and a temperature analog signal detected by the temperature sensing unit, and then transmitting the switched air pressure analog signal or the switched temperature analog signal to the analog-to-digital conversion module in turn, and the analog-to-digital conversion module is capable of converting the temperature analog signal into a temperature digital signal.

2. The electronic vaporizing device chip with an air pressure sensing unit according to claim 1, wherein the control unit is inactive in a standby state, the air pressure sensing unit is configured to have a patrol detection for the air pressure, and the control unit immediately enters a working state once a change of the air pressure is detected.

3. The electronic vaporizing device chip with an air pressure sensing unit according to claim 1, wherein the plurality of pins comprise at least a power supply pin, a ground pin, an activation signal output pin, an internal data output pin, an external data input pin, a clock signal pin, and an enabling signal input pin, the activation signal output pin serves to output an activation signal generated by the control unit to an external controller so as to activate the electronic vaporizing device, the internal data output pin, the external data input pin, and the clock signal pin are connected to the communication interface module, the internal data output pin serves to output internal data to the external controller, and the external data input pin serves to input external data, the clock signal pin serves to input a clock signal to read and write data, and the enabling signal input pin serves to input an enabling signal to control an output of the internal data and an input of the external data.

4. The electronic vaporizing device chip with an air pressure sensing unit according to claim 3, wherein an air pressure change threshold value is preset in the memory, and the control unit is configured to generate the activation signal in a condition that the control unit determines that a change value of the air pressure digital signal reaches the preset air pressure change threshold value.

5. The electronic vaporizing device chip with an air pressure sensing unit according to claim 1, wherein the control unit further comprises an output control module electrically connected with the data processing and calibration module, and the output control module is configured to generate a PWM output control signal based on the air flow quantity digital signal for controlling power of the electronic vaporizing device.

6. The electronic vaporizing device chip with an air pressure sensing unit according to claim 5, wherein the pins comprise a PWM output control signal pin serves to output the PWM output control signal generated by the output control module.

7. The electronic vaporizing device chip with an air pressure sensing unit according to claim 1, wherein the control unit further comprises an on-chip oscillator for providing a frequency signal for the control unit during working.

8. The electronic vaporizing device chip with an air pressure sensing unit according to claim 1, wherein the control unit further comprises a real-time clock module for timing.

9. A working method of an electronic vaporizing device chip with an air pressure sensing unit, wherein the method comprises steps of:
(1) initializing a relevant parameter when the chip is powered;
(2) activating an air pressure sensing unit and a temperature sensing unit when the chip is in a standby state, to simultaneously go to steps (3) and (4);
(3) determining whether the air pressure sensing unit detects a change of an air pressure analog signal of the electronic vaporizing device, if yes, go to step (5), if no, return to step (2);
(4) detecting a temperature analog signal of an internal temperature of the chip by the temperature sensing unit;
(5) switching the air pressure analog signal and the temperature analog signal in turn by a circuit switching module, if being switched to the air pressure analog signal, go to step (6); if being switched to the temperature analog signal, go to step (12);
(6) amplifying the air pressure analog signal by a signal amplification module and converting the amplified air pressure analog signal into an air pressure digital signal by an analog-to-digital conversion module;
(7) processing and calibrating the air pressure digital signal by a data processing and calibration module and converting the processed and calibrated air pressure digital signal into a corresponding air flow quantity digital signal;
(8) by means of a control logic module, determining whether a change value of the air pressure digital signal reaches a set air pressure change threshold value, if yes, simultaneously go to steps (9), (10), and (12), if no, return to step (2);
(9) generating an activation signal by a control unit and outputting the activation signal to an external controller through an activation signal output terminal to activate the electronic vaporizing device;
(10) determining whether the air flow quantity digital signal reaches a set minimum air flow quantity threshold value, if yes, go to next step, if no, return to step (2);
(11) generating and outputting a PWM output control signal by an output control module, then returning to step (2);
(12) determining whether an enabling signal input control circuit receives an enabling signal and whether a communication interface module receives a data transmission request signal, if both yes, go to next step, if no, return to step (2);
(13) outputting the air flow quantity digital signal to the external controller through an internal data output pin by the communication interface module, then returning to step (2);
(14) amplifying the temperature analog signal by the signal amplification module and converting the amplified temperature analog signal into a temperature digital signal by the analog-to-digital conversion module;
(15) by means of the control logic module, determining whether the temperature digital signal reaches a set temperature threshold value, if yes, go to next step, if no, return to step (2); and
(16) inactivating the chip and providing high temperature or low temperature protection.

## Patentansprüche

1. Elektronischer Verdampferchip mit einer Luftdrucksensoreinheit, umfassend eine Luftdrucksensoreinheit, eine Steuereinheit, eine Vielzahl von Hilfswiderständen, einen Kondensator und eine Vielzahl von Pins, wobei die Luftdrucksensoreinheit, die Steuereinheit, die Vielzahl von Hilfswiderständen, der Kondensator und die Vielzahl von Pins elektrisch verbunden sind, **dadurch gekennzeichnet, dass** die Steuereinheit einen Speicher, ein Steuerlogikmodul, ein Ansteuerungsmodul für die Luftdrucksensoreinheit, ein Signalverstärkungsmodul, ein Analog-digital-Wandlermodul, ein Datenverarbeitungs- und Kalibriermodul, ein Kommunikationsschnittstellenmodul und eine Freigabesignaleingangs-Steuerschaltung umfasst, die Luftdrucksensoreinheit ausgelegt ist, einen Luftdruck zu erkennen, der im Inneren des elektronischen Verdampfers während eines Saugvorgangs erzeugt wird, der Speicher ausgelegt ist, einen relevanten Parameter zu speichern, das Steuerlogikmodul zur Logiksteuerung einer internen Schaltung der Steuereinheit ausgelegt ist, das Ansteuerungsmodul für die Luftdrucksensoreinheit ausgelegt ist, die Luftdrucksensoreinheit zum Betrieb anzusteuern, das Signalverstärkungsmodul ausgelegt ist, ein von der Luftdrucksensoreinheit erkanntes Luftdruck-Analogsignal zu verstärken und dann an das Analog-digital-Wandlermodul zu übertragen, das Analog-digital-Wandlermodul ausgelegt ist, das Luftdruck-Analogsignal in ein Luftdruck-Digitalsignal umzuwandeln, das Datenverarbeitungs- und Kalibriermodul ausgelegt ist, das Luftdruck-Digitalsignal zu verarbeiten und zu kalibrieren und danach das Luftdruck-Digitalsignal in ein Luftstrommengen-Digitalsignal umzuwandeln, das Kommunikationsschnittstellenmodul für eine Kommunikationsverbindung mit einer externen Komponente ausgelegt ist und die Freigabesignaleingangs-Steuerschaltung ausgelegt ist, ein externes Freigabesignal zu empfangen und den Betrieb des Kommunikationsschnittstellenmoduls zu steuern, und der elektronische Verdampferchip ferner eine Temperatursensoreinheit zum Erkennen einer Temperatur des elektronischen Verdampferchips umfasst, wobei die Steuereinheit ferner ein Schaltungswechselmodul zum Umschalten zwischen dem von der Luftdrucksensoreinheit erkannten Luftdruck-Analogsignal und einem von der Temperatursensoreinheit erkannten Temperatur-Analogsignal und nachfolgendem abwechselnden Übertragen des umgeschalteten Luftdruck-Analogsignals oder des umgeschalteten Temperatur-Analogsignals an das Analog-digital-Wandlermodul umfasst, und das Analog-digital-Wandlermodul fähig ist, das Temperatur-Analogsignal in ein Temperatur-Digitalsignal umzuwandeln.

2. Elektronischer Verdampferchip mit einer Luftdrucksensoreinheit nach Anspruch 1, wobei die Steuereinheit in einem Bereitschaftszustand inaktiv ist, die Luftdrucksensoreinheit ausgelegt ist, den Luftdruck überwachend zu erkennen, und die Steuereinheit sofort in einen Betriebszustand tritt, sobald eine Änderung des Luftdrucks erkannt wird.

3. Elektronischer Verdampferchip mit einer Luftdrucksensoreinheit nach Anspruch 1, wobei die Vielzahl von Pins mindestens einen Leistungsversorgungspin, einen Erdungspin, einen Pin zur Ausgabe von Aktivierungssignalen, einen Pin zur Ausgabe von internen Daten, einen Pin zur Eingabe von externen Daten, einen Taktsignalpin und einen Pin zur Eingabe von Freigabesignalen umfasst, wobei der Pin zur Ausgabe von Aktivierungssignalen dazu dient, ein von der Steuereinheit erzeugtes Aktivierungssignal an eine externe Steuerung auszugeben, um den elektronischen Verdampfer zu aktivieren, wobei der Pin zur Ausgabe von internen Daten, der Pin zur Eingabe von externen Daten und der Taktsignalpin mit dem Kommunikationsschnittstellenmodul verbunden sind, der Pin zur Ausgabe von internen Daten dazu dient, interne Daten an die externe Steuerung auszugeben, und der Pin zur Eingabe von externen Daten dazu dient, externe Daten einzugeben, der Taktsignalpin dazu dient, ein Taktsignal zum Lesen und Schreiben von Daten einzugeben, und der Pin zur Eingabe von Freigabesignalen dazu dient, ein Freigabesignal einzugeben, um eine Ausgabe der internen Daten und eine Eingabe der externen Daten zu steuern.

4. Elektronischer Verdampferchip mit einer Luftdrucksensoreinheit nach Anspruch 3, wobei ein Luftdruckänderungsschwellenwert im Speicher voreingestellt ist und die Steuereinheit ausgelegt ist, das Aktivierungssignal unter der Bedingung zu erzeugen, dass die Steuereinheit ermittelt, dass ein Änderungswert des Luftdruck-Digitalsignals den voreingestellten Luftdruckänderungsschwellenwert erreicht.

5. Elektronischer Verdampferchip mit einer Luftdrucksensoreinheit nach Anspruch 1, wobei die Steuereinheit ferner ein Ausgangssteuermodul umfasst, das elektrisch mit dem Datenverarbeitungs- und Kalibriermodul verbunden ist, und das Ausgangssteuermodul ausgelegt ist, ein PWM-Ausgangssteuersignal auf Grundlage des Luftstrommengen-Digitalsignals zum Steuern einer Leistung des elektronischen Verdampfers zu erzeugen.

6. Elektronischer Verdampferchip mit einer Luftdrucksensoreinheit nach Anspruch 5, wobei die Pins einen PWM-Ausgangssteuersignalpin umfassen, der dazu dient, das vom Ausgangssteuermodul erzeugte PWM-Ausgangssteuersignal auszugeben.

7. Elektronischer Verdampferchip mit einer Luftdrucksensoreinheit nach Anspruch 1, wobei die Steuereinheit ferner einen chipinternen Oszillator zum Bereitstellen eines Frequenzsignals für die Steuereinheit während des Betriebs umfasst.

8. Elektronischer Verdampferchip mit einer Luftdrucksensoreinheit nach Anspruch 1, wobei die Steuereinheit ferner ein Echtzeittaktmodul zur Zeitgebung umfasst.

9. Betriebsverfahren für einen elektronischen Verdampferchip mit einer Luftdrucksensoreinheit, wobei das Verfahren die Schritte umfasst zum:
(1) Initialisieren eines relevanten Parameters, wenn der Chip mit Leistung versorgt wird;
(2) Aktivieren einer Luftdrucksensoreinheit und einer Temperatursensoreinheit, wenn sich der Chip in einem Bereitschaftszustand befindet, um gleichzeitig zu Schritt (3) und Schritt (4) zu gehen;
(3) Ermitteln, ob die Luftdrucksensoreinheit eine Änderung eines Luftdruck-Analogsignals des elektronischen Verdampfers erkennt, falls ja, Gehen zu Schritt (5), falls nein, Zurückkehren zu Schritt (2);
(4) Erkennen eines Temperatur-Analogsignals einer internen Temperatur des Chips durch die Temperatursensoreinheit;
(5) abwechselndes Umschalten zwischen dem Luftdruck-Analogsignal und dem Temperatur-Analogsignal durch ein Schaltungswechselmodul, falls zum Luftdruck-Analogsignal umgeschaltet ist, Gehen zu Schritt (6); falls zum Temperatur-Analogsignal umgeschaltet ist, Gehen zu Schritt (12);
(6) Verstärken des Luftdruck-Analogsignals durch ein Signalverstärkungsmodul und Umwandeln des verstärkten Luftdruck-Analogsignals in ein Luftdruck-Digitalsignal durch ein Analog-digital-Wandlermodul;
(7) Verarbeiten und Kalibrieren des Luftdruck-Digitalsignals durch ein Datenverarbeitungs- und Kalibriermodul und Umwandeln des verarbeiteten und kalibrierten Luftdruck-Digitalsignals in ein entsprechendes Luftstrommengen-Digitalsignal;
(8) mithilfe eines Steuerlogikmoduls, Ermitteln, ob ein Änderungswert des Luftdruck-Digitalsignals einen eingestellten Luftdruckänderungsschwellenwert erreicht, falls ja, gleichzeitiges Gehen zu Schritt (9), (10) und (12), falls nein, Zurückkehren zu Schritt (2);
(9) Erzeugen eines Aktivierungssignals durch eine Steuereinheit und Ausgeben des Aktivierungssignals über einen Aktivierungssignal-Ausgangsanschluss an eine externe Steuerung, um den elektronischen Verdampfer zu aktivieren;
(10) Ermitteln, ob das Luftstrommengen-Digitalsignal einen eingestellten minimalen Luftstrommengenschwellenwert erreicht, falls ja, Gehen zum nächsten Schritt, falls nein, Zurückkehren zu Schritt (2);
(11) Erzeugen und Ausgeben eines PWM-Ausgangssteuersignals durch ein Ausgangssteuermodul, danach Zurückkehren zu Schritt (2);
(12) Ermitteln, ob eine Freigabesignaleingangs-Steuerschaltung ein Freigabesignal empfängt und ob ein Kommunikationsschnittstellenmodul ein Datenübertragungs-Anforderungssignal empfängt, falls beide ja, Gehen zum nächsten Schritt, falls nein, Zurückkehren zu Schritt (2);
(13) Ausgeben des Luftstrommengen-Digitalsignals über einen Pin zur Ausgabe interner Daten durch das Kommunikationsschnittstellenmodul an die externe Steuerung, danach Zurückkehren zu Schritt (2);
(14) Verstärken des Temperatur-Analogsignals durch das Signalverstärkungsmodul und Umwandeln des verstärkten Temperatur-Analogsignals in ein Temperatur-Digitalsignal durch das Analog-digital-Wandlermodul;
(15) mithilfe eines Steuerlogikmoduls, Ermitteln, ob das Temperatur-Digitalsignal einen eingestellten Temperaturschwellenwert erreicht, falls ja, Gehen zum nächsten Schritt, falls nein, Zurückkehren zu Schritt (2); und
(16) Deaktivieren des Chips und Bereitstellen eines Hochtemperatur- oder Tieftemperaturschutzes.

## Revendications

1. Puce pour dispositif de vaporisation électronique avec une unité de détection de pression de l'air, comprenant une unité de détection de pression de l'air, une unité de commande, une pluralité de résistances auxiliaires, un condensateur et une pluralité de broches, dans laquelle l'unité de détection de pression de l'air, l'unité de commande, la pluralité de résistances auxiliaires, le condensateur et la pluralité de broches sont connectés électriquement, **caractérisée en ce que** l'unité de commande comprend une mémoire, un module de logique de commande, un module d'entraînement d'unité de détection de pression de l'air, un module d'amplification de signal, un module de conversion analogique-numérique, un module de traitement de données et d'étalonnage, un module d'interface de communication et un circuit de commande d'entrée de signal d'activation, l'unité de détection de pression de l'air est configurée pour détecter une pression de l'air générée à l'intérieur du dispositif de vaporisation électronique au cours de l'aspiration, la mémoire est configurée pour stocker un paramètre pertinent, le module de logique de commande est configuré pour une commande logique d'un circuit interne de l'unité de commande, le module d'entraînement d'unité de détection de pression de l'air est configuré pour entraîner l'unité de détection de pression de l'air à fonctionner, le module d'amplification de signal est configuré pour amplifier, puis transmettre un signal analogique de pression de l'air détecté par l'unité de détection de pression de l'air au module de conversion analogique-numérique, le module de conversion analogique-numérique est configuré pour convertir le signal analogique de pression de l'air en un signal numérique de pression de l'air, le module de traitement de données et d'étalonnage est configuré pour traiter et étalonner le signal numérique de pression de l'air, puis convertir le signal numérique de pression de l'air en un signal numérique de quantité d'écoulement d'air, le module d'interface de communication est configuré pour une connexion de communication avec un composant externe, et le circuit de commande d'entrée de signal d'activation est configuré pour recevoir un signal d'activation externe et commander le module d'interface de communication de fonctionner, et la puce pour dispositif de vaporisation électronique comprend en outre une unité de détection de température pour détecter la température de la puce pour dispositif de vaporisation électronique, dans laquelle l'unité de commande comprend en outre un module de commutation de circuit pour commuter le signal analogique de pression de l'air détecté par l'unité de détection de pression de l'air et un signal analogique de température détecté par l'unité de détection de température, puis transmettre le signal analogique de pression de l'air commuté ou le signal analogique de température commuté au module de conversion analogique-numérique l'un après l'autre, et le module de conversion analogique-numérique est capable de convertir le signal analogique de température en un signal numérique de température.

2. Puce pour dispositif de vaporisation électronique avec une unité de détection de pression de l'air selon la revendication 1, dans laquelle l'unité de commande est inactive dans un état d'attente, l'unité de détection de pression de l'air est configurée pour avoir une détection de patrouille pour la pression de l'air, et l'unité de commande entre immédiatement dans un état de fonctionnement une fois qu'un changement de la pression de l'air est détecté.

3. Puce pour dispositif de vaporisation électronique avec une unité de détection de pression de l'air selon la revendication 1, dans laquelle la pluralité de broches comprennent au moins une broche d'alimentation électrique, une broche de mise à la terre, une broche de sortie de signal d'activation, une broche de sortie de données internes, une broche d'entrée de données externes, une broche de signal d'horloge et une broche d'entrée de signal d'activation, la broche de sortie de signal d'activation sert à émettre un signal d'activation généré par l'unité de commande vers un contrôleur externe de manière à activer le dispositif de vaporisation électronique, la broche de sortie de données internes, la broche d'entrée de données externes et la broche de signal d'horloge sont connectées au module d'interface de communication, la broche de sortie de données internes sert à émettre des données internes vers le contrôleur externe, et la broche d'entrée de données externes sert à entrer des données externes, la broche de signal d'horloge sert à entrer un signal d'horloge pour lire et écrire des données, et la broche d'entrée de signal d'activation sert à entrer un signal d'activation pour commander une sortie des données internes et une entrée des données externes.

4. Puce pour dispositif de vaporisation électronique avec une unité de détection de pression de l'air selon la revendication 3, dans laquelle une valeur seuil de changement de pression de l'air est prédéfinie dans la mémoire, et l'unité de commande est configurée pour générer le signal d'activation dans une condition dans laquelle l'unité de commande détermine qu'une valeur de changement du signal numérique de pression de l'air atteint la valeur seuil de changement de pression de l'air prédéfinie.

5. Puce pour dispositif de vaporisation électronique avec une unité de détection de pression de l'air selon la revendication 1, dans laquelle l'unité de commande comprend en outre un module de commande de sortie connecté électriquement au module de traitement de données et d'étalonnage, et le module de commande de sortie est configuré pour générer un signal de commande de sortie à modulation d'impulsions en durée basé sur le signal numérique de quantité d'écoulement d'air pour commander la puissance du dispositif de vaporisation électronique.

6. Puce pour dispositif de vaporisation électronique avec une unité de détection de pression de l'air selon la revendication 5, dans laquelle les broches comprennent une broche de signal de commande de sortie à modulation d'impulsions en durée qui sert à émettre le signal de commande de sortie à modulation d'impulsions en durée généré par le module de commande de sortie.

7. Puce pour dispositif de vaporisation électronique avec une unité de détection de pression de l'air selon la revendication 1, dans laquelle l'unité de commande comprend en outre un oscillateur intégré pour fournir un signal de fréquence pour l'unité de commande au cours du fonctionnement.

8. Puce pour dispositif de vaporisation électronique avec une unité de détection de pression de l'air selon la revendication 1, dans laquelle l'unité de commande comprend en outre un module d'horloge en temps réel pour la minuterie.

9. Procédé de fonctionnement d'une puce pour dispositif de vaporisation électronique avec une unité de détection de pression de l'air, dans lequel le procédé comprend les étapes consistant à :
(1) initialiser un paramètre pertinent lorsque la puce est alimentée ;
(2) activer une unité de détection de pression de l'air et une unité de détection de température lorsque la puce est dans un état d'attente pour aller simultanément aux étapes (3) et (4) ;
(3) déterminer si l'unité de détection de pression de l'air détecte un changement d'un signal analogique de pression de l'air du dispositif de vaporisation électronique, si c'est le cas, aller à l'étape (5), sinon, retourner à l'étape (2) ;
(4) détecter un signal analogique de température d'une température interne de la puce par l'unité de détection de température ;
(5) commuter le signal analogique de pression de l'air et le signal analogique de température l'un après l'autre par un module de commutation de circuit, en cas de commutation au signal analogique de pression de l'air, aller à l'étape (6) ; en cas de commutation au signal analogique de température, aller à l'étape (12) ;
(6) amplifier le signal analogique de pression de l'air par un module d'amplification de signal et convertir le signal analogique de pression de l'air amplifié en un signal numérique de pression de l'air par un module de conversion analogique-numérique ;
(7) traiter et étalonner le signal numérique de pression de l'air par un module de traitement de données et d'étalonnage, et convertir le signal numérique de pression de l'air traité et étalonné en un signal numérique de quantité d'écoulement d'air correspondant ;
(8) au moyen d'un module de logique de commande, déterminer si une valeur de changement du signal numérique de pression de l'air atteint une valeur seuil de changement de pression de l'air prédéfinie, si c'est le cas, aller simultanément aux étapes (9), (10) et (12), sinon, retourner à l'étape (2) ;
(9) générer un signal d'activation par une unité de commande et émettre le signal d'activation vers un contrôleur externe par une borne de sortie de signal d'activation pour activer le dispositif de vaporisation électronique ;
(10) déterminer si le signal numérique de quantité d'écoulement d'air atteint une valeur seuil de quantité d'écoulement d'air minimale définie, si c'est le cas, aller l'étape suivante, sinon, retourner à l'étape (2) ;
(11) générer et émettre un signal de commande de sortie à modulation d'impulsions en durée par un module de commande de sortie, puis retourner à l'étape (2) ;
(12) déterminer si un circuit de commande d'entrée de signal d'activation reçoit un signal d'activation et si un module d'interface de communication reçoit un signal de requête de transmission de données, si c'est le cas pour les deux, aller l'étape suivante, sinon, retourner à l'étape (2) ;
(13) émettre le signal numérique de quantité d'écoulement d'air vers le contrôleur externe à travers une broche de sortie de données internes par le module d'interface de communication, puis retourner à l'étape (2) ;
(14) amplifier le signal analogique de température par le module d'amplification de signal et convertir le signal analogique de température amplifié en un signal numérique de température par le module de conversion analogique-numérique ;
(15) au moyen du module de logique de commande, déterminer si le signal numérique de température atteint une valeur seuil de température définie, si c'est le cas, aller à l'étape suivante, sinon, retourner à l'étape (2) ; et
(16) désactiver la puce et fournir une protection haute température ou basse température.
